# EUROPEAN PATENT APPLICATION

(11) **EP 3 320 832 A1**
(43) Date of publication of application: **16.05.2018**
(21) Application number: 17201180.1
(22) Date of filing: 10.11.2017
(51) Int. Cl.: A61B 5/00, G06F 1/16, G06F 1/32

(54) **ELECTRONIC APPARATUS AND OPERATING METHOD THEREOF**

(30) Priority: 10.11.2016 KR 20160149181
(71) Applicant: Samsung Electronics Co., Ltd., Gyeonggi-do 16677 (KR)
(72) Inventor: Seo, Yunhwa, Suwon-si (KR); Kim, Narin, Seoul (KR); Lee, Byungjun, Uiwang-si (KR); Kang, Jeong Gwan, Hwaseong-si (KR)
(74) Representative: Nederlandsch Octrooibureau

(57) **Abstract**

An electronic apparatus and an operating method are provided. The electronic apparatus includes a motion sensor, an optical sensor, a memory, and a processor. The processor determines whether the electronic apparatus is worn on a user's body using at least one of the motion sensor and the optical sensor. The processor obtains sensor data according to a motion of the electronic apparatus using at least one of the motion sensor and the optical sensor when determining whether the electronic apparatus is worn on the user's body. In addition, the processor compares the obtained data with at least one reference information stored in the memory, determines whether the electronic apparatus is normally worn based on the comparison, and performs at least one operation based on the determination.

## Description

### BACKGROUND

### 1. Field of Disclosure

The present disclosure relates generally to an electronic apparatus which can be fitted into a position on the body of a user, and an operating method thereof

### 2. Description of Related Art

Electronic devices that perform the same or new functions, are becoming smaller, slimmer, and more portable. Such electronic devices are generally carried in a user's pocket, but can be worn on a wrist, a head portion, or an arm of the user.

Further, electronic devices may be fitted into a position on the user's body. For example, the electronic device can include wireless earphones capable of being inserted and fitted in a user's ears.

Body-fittable electronic devices can include sensors such as an inertial sensor, a photoelectric sensor, an electromagnetic sensor, etc. Such sensors may acquire inaccurate sensor data due to abnormal wearing of the electronic device. For example, the abnormal wearing can indicate that the electronic device is not normally fitted into the user's body, or is worn on the user's body in an abnormal direction. When the electronic device is abnormally worn on the user's body, an axis of an inertial sensor is changed or a photoelectric sensor is not fitted to the user's body, and thus normal activity information cannot be measured.

### SUMMARY

An aspect of the present disclosure is to provide an electronic apparatus, and a method thereof, for determining whether the electronic apparatus is normally worn based on a motion of the electronic apparatus.

Another aspect of the present disclosure provides an electronic apparatus, and method thereof, for outputting guide information regarding abnormal wearing in response to abnormal wearing of the electronic apparatus on a user's body.

Another aspect of the present disclosure provides an electronic apparatus, and method thereof, for changing a setting of the electronic apparatus in response to abnormal wearing of the electronic apparatus on a user's body.

According to an aspect of the present disclosure, an electronic apparatus includes a motion sensor, an optical sensor, a memory, and a processor. The processor can determine whether the electronic apparatus is worn on a user's body using at least one of the motion sensor and the optical sensor. When determining whether the electronic apparatus is worn on the user's body, the processor can obtain sensor data according to a motion of the electronic apparatus using at least one of the motion sensor and the optical sensor. The processor can compare the obtained data with at least one reference information stored in the memory, determine whether the electronic apparatus is normally worn based on the comparison, and perform at least one operation based on the determination.

According to an aspect of the present disclosure, a method for operating an electronic apparatus includes determining whether the electronic apparatus is worn on a user's body using at least one of a motion sensor and an optical sensor, obtaining sensor data according to a motion of the electronic apparatus using at least one of the motion sensor and the optical sensor when determining whether the electronic apparatus is worn on the user's body, comparing the obtained data with at least one reference information stored, determining whether the electronic apparatus is normally worn based on the comparison, and performing at least one operation based on the determination.

According to another aspect of the present disclosure, a computer-readable recording medium can store a program for determining whether an electronic apparatus is worn on a user's body using at least one of a motion sensor and an optical sensor, obtaining sensor data according to a motion of the electronic apparatus using at least one of the motion sensor and the optical sensor when determining whether the electronic apparatus is worn on the user's body, comparing the obtained data with at least one reference information stored, determining whether the electronic apparatus is normally worn based on the comparison, and performing at least one operation based on the determination.

### BRIEF DESCRIPTION OF THE DRAWINGS

The above and other aspects, features, and advantages of the present disclosure will be more apparent from the following detailed description taken in conjunction with the accompanying drawings, in which:
FIG. 1 is a diagram of a set of electronic apparatuses according to embodiments of the present disclosure;
FIG. 2 is a diagram of an electronic apparatus in a network according to embodiments of the present disclosure;
FIG. 3 is a block diagram of an electronic apparatus according to embodiments of the present disclosure;
FIG. 4 is a block diagram of a program module according to embodiments of the present disclosure;
FIG. 5 is a flowchart for determining a normal wearing state in an electronic apparatus according to embodiments of the present disclosure;
FIG. 6 is a flowchart for determining a wearing state of an electronic apparatus according to embodiments of the present disclosure;
FIG. 7 is a flowchart for determining wearing of an electronic apparatus according to various embodiments of the present disclosure;
FIGS. 8A and 8B are diagrams for determining wearing according to embodiments of the present disclosure;
FIG. 9 is a flowchart for determining a wearing state of an electronic apparatus according to embodiments of the present disclosure;
FIGS. 10A through 10D are graphs of sensor data for determining a wearing state according to embodiments of the present disclosure;
FIG. 11 is a flowchart for abnormal wearing of an electronic apparatus according to embodiments of the present disclosure; and
FIG. 12 is a graph of sensor data in relation to wearing state change of an electronic apparatus according to embodiments of the present disclosure.

### DETAILED DESCRIPTION

Embodiments of the present disclosure are described in detail with reference to the accompanying drawings. The same or similar components may be designated by the same or similar reference numerals although they are illustrated in different drawings. Detailed descriptions of constructions or processes known in the art may be omitted to avoid obscuring the subject matter of the present disclosure. The terms used herein are defined in consideration of functions of the present disclosure and may vary depending on a user's or an operator's intention and usage. Therefore, the terms used herein should be understood based on the descriptions made herein.

It is to be understood that the singular forms "a," "an," and "the" include plural referents unless the context clearly dictates otherwise. In the present disclosure, the terms "A or B," "at least one of A and B," or "one or more of A and B" may include all possible combinations of A and B. Expressions such as "first," "second," "primarily," or "secondary," as used herein, may represent various elements regardless of order and/or importance and are used to distinguish one element from another element without limiting the elements. When it is described that an element (such as a first element) is "operatively coupled" or "communicatively coupled" to, or "connected" to, another element (such as a second element), the element can be directly connected to the other element or can be connected through another element (such as a third element).

An expression "configured to" or "set to" used in the present disclosure may be used interchangeably with, for example, "suitable for," "having the capacity to," "designed to," "adapted to," "made to," or "capable of'. A term "configured to" or "set to" does not only mean "specifically designed to" by hardware. Alternatively, in some situations, the expression "apparatus configured to" may mean that the apparatus can operate together with another apparatus or component. For example, a phrase "a processor configured to perform A, B, and C" or "a processor set to perform A, B, and C" may be a generic-purpose processor, such as a central processing unit (CPU) or an application processor (AP), that can perform a corresponding operation by executing at least one software program stored in an embedded processor for performing a corresponding operation or in a memory device.

An electronic device may be embodied as, for example, at least one of a smart phone, a tablet personal computer (PC), a mobile phone, a video phone, an e-book reader, a desktop PC, a laptop PC, a netbook computer, a workstation, a server, a personal digital assistant (PDA), a portable multimedia player (PMP), an MPEG 3 (MP3) player, a medical device, a camera, and a wearable device. The wearable device can include at least one of an accessory type (e.g., a watch, a ring, a bracelet, an ankle bracelet, a necklace, glasses, a contact lens, or a head-mounted-device (HMD)), a fabric or clothing embedded type (e.g., electronic garments), a body attachable type (e.g., a skin pad or a tattoo), and an implantable circuit. The electronic device may be embodied as at least one of, for example, a television, a digital versatile disc (DVD) player, an audio device, a refrigerator, an air-conditioner, a cleaner, an oven, a microwave oven, a washing machine, an air cleaner, a set-top box, a home automation control panel, a security control panel, a media box (e.g., Samsung HomeSync™, Apple TV™, or Google TV™), a game console (e.g., Xbox™, PlayStation™), an electronic dictionary, an electronic key, a camcorder, and an electronic frame.

The electronic device may be embodied as at least one of various medical devices (e.g., various portable medical measuring devices (a blood sugar measuring device, a heartbeat measuring device, a blood pressure measuring device, or a body temperature measuring device), a magnetic resonance angiography (MRA) device, a magnetic resonance imaging (MRI) device, a computed tomography (CT) device, a scanning machine, and an ultrasonic wave device), a navigation device, a global navigation satellite system (GNSS), an event data recorder (EDR), a flight data recorder (FDR), a vehicle infotainment device, electronic equipment for ship (such as, a navigation device for ship and gyro compass), avionics, a security device, a head unit for a vehicle, an industrial or home robot, a drone, an automated teller machine (ATM) of a financial institution, a point of sales (POS) device of a store, and an Internet of Things (IoT) device (e.g., a light bulb, various sensors, a sprinkler device, a fire alarm, a thermostat, a street light, a toaster, sports equipment, a hot water tank, a heater, and a boiler).

According to an embodiment, the electronic device may be at least one of a portion of furniture, building/construction or vehicle, an electronic board, an electronic signature receiving device, a projector, and various measuring devices (e.g., devices for measuring water, electricity, gas, or electromagnetic waves). The electronic device can be a flexible electronic device or a combination of two or more of the foregoing devices. An electronic device is not limited to the foregoing devices and may be a newly developed electronic device. The term "user" can refer to a person using an electronic device or a device using an electronic device (e.g., an artificial intelligence electronic device).

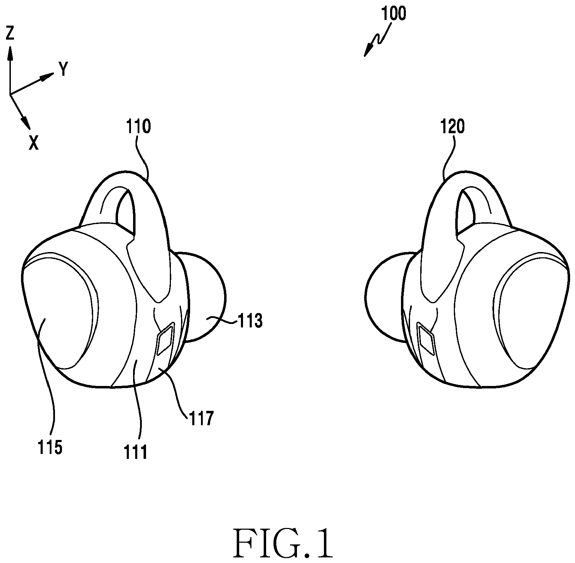

FIG. 1 is a diagram of a set of electronic apparatuses 100 according to embodiments of the present disclosure.

The set of electronic apparatuses 100 can include a first electronic apparatus 110 and a second electronic apparatus 120 of an ear wearable type which can be worn on, fitted in, or positioned on, a body or part (e.g., ears) of a user. In a three-dimensional X/Y/Z orthogonal coordinate system, the Z axis can indicate a depth (e.g., thickness), the X axis can indicate a width, and the Y axis can indicate a height of a main body of the first electronic apparatus 110 and/or the second electronic apparatus 120.

The first electronic apparatus 110 and/or the second electronic apparatus 120 can be a communication electronic device including a speaker and a microphone. The main body 110 of the first electronic apparatus 110 and/or the second electronic apparatus 120 can include a tip 113, at least one key button 115, and at least one sensor 117.

The tip 113 can aid in fitting the first electronic apparatus 110 and/or the second electronic apparatus 120 into a part of a user's body. For example, the tip 113 can be elastic and substantially cylindrical. The tip 113 can be attached to and detached from the main body 111 of the first electronic apparatus 110 and/or the second electronic apparatus 120.

The at least one key button 115 can generate a control signal for controlling the first electronic apparatus 110 and/or the second electronic apparatus 120. For example, the control signal can include a signal to power on, power off, control volume, start data play, pause play, stop play, control play speed, as well as other signals. The at least one key button 150 can include a physical button, an optical key, or a keypad.

The at least one sensor 117 can include at least one of an acceleration sensor, a gyro sensor, a geomagnetic sensor, a heart rate sensor, a proximity sensor, an illumination sensor, a galvanic skin response (GSR) sensor, an electrocardiogram (ECG) sensor, an electromyography (EMG) sensor, a blood glucose sensor, a blood pressure sensor, a pressure sensor, and a temperature sensor. Additionally, the at least one sensor 117 may include sensors for detecting a value to determine whether the first electronic apparatus 110 and/or the second electronic apparatus 120 is worn on the body. Some sensors (e.g., the heart rate sensor, the proximity sensor, the illumination sensor, the GSR sensor, the ECG sensor, the EMG sensor, the blood glucose sensor, the blood pressure sensor, the pressure sensor, or the temperature sensor) can be disposed closely to at least part of the body when the first electronic apparatus 110 and/or the second electronic apparatus 120 is worn on, fitted in, or positioned on the user's body.

The first electronic apparatus 110 and/or the second electronic apparatus 120 can include at least one processor for controlling a component of the first electronic apparatus 110 and/or the second electronic apparatus 120 (e.g., the at least one key button 115, the at least one sensor 117, etc.). Also, the first electronic apparatus 110 and/or the second electronic apparatus 120 can include a memory for storing commands and data regarding at least one component of the first electronic apparatus 110 and/or the second electronic apparatus 120. Further, the first electronic apparatus 110 and/or the second electronic apparatus 120 may include a battery for supplying power to the first electronic apparatus 110 and/or the second electronic apparatus 120.

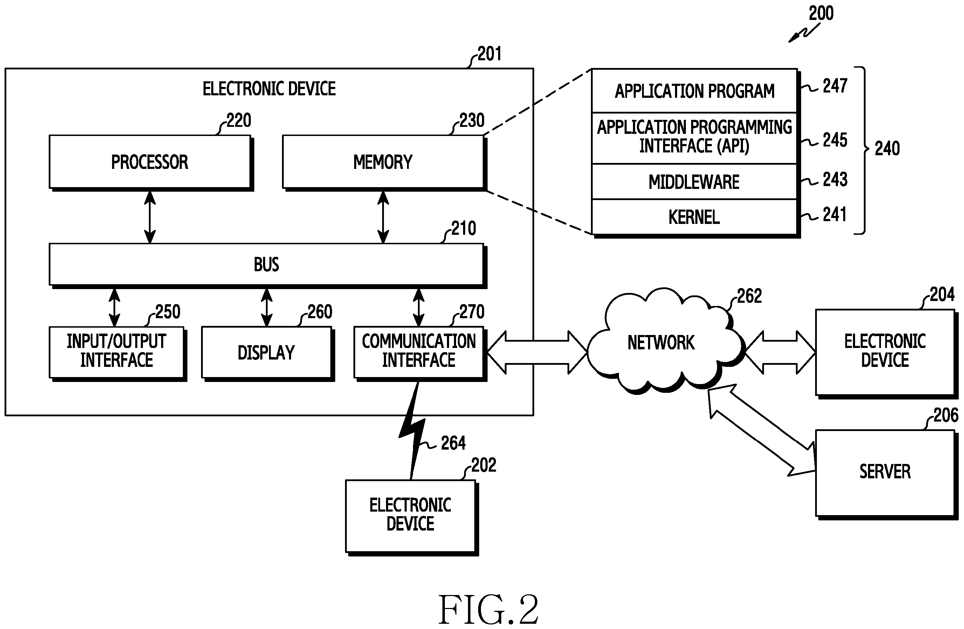

FIG. 2 is a diagram of an electronic apparatus in a network according to embodiments of the present disclosure.

An electronic device 201 may reside in a network environment 200. The electronic device 201 may include at least part of the first electronic apparatus 110 and/or the second electronic apparatus 120 illustrated in FIG. 1. The electronic device 201 can include a bus 210, a processor 220, a memory 230, an input/output interface 250, a display 260, and a communication interface 270. The electronic device 201 may be provided without at least one of the aforementioned components, or may include at least one additional component.

The bus 210 can include a circuit for connecting components 220 through 270 and delivering communication signals (e.g., control messages or data) among them. The processor 220 can include one or more of a CPU, an AP, and a communication processor (CP). The processor 220, for example, can perform an operation or process data with respect to the control of, or communication with, at least another component of the electronic device 201.

The processor 220 can determine a wearing state of the electronic apparatus 201. For example, the processor 220 can determine whether the electronic apparatus 201 can be worn on the body based on at least one sensor.

The processor 220 can determine the wearing state based on at least one first sensor which can determine a motion of the electronic apparatus 201. The first sensor can include an inertial, or motion, sensor (e.g., an acceleration sensor, a geomagnetic sensor, a gyro sensor). For example, when detecting the motion (or a motion change) of the electronic apparatus 201 corresponding to a certain direction or speed, the processor 220 can determine that a context for wearing the electronic apparatus 201 on the body (e.g., a context where the user moves the electronic apparatus 201 to wear it) is detected. When not detecting the motion (or the motion change) of the electronic apparatus 201 corresponding to the certain direction or speed, the processor 220 can determine that the context for wearing the electronic apparatus 201 on the body is not detected.

Upon determining the wearing state of the electronic apparatus 201, the processor 220 can determine whether the electronic apparatus 201 is worn on the body of a user based on at least one second sensor which can determine proximity to a target. The second sensor can include a photoelectric sensor (e.g., an infrared sensor, a bio sensor), and an electromagnetic sensor (e.g., a grip sensor, an EMG sensor). For example, when detecting the target within a certain distance from the electronic apparatus 201, the processor 220 can determine that the electronic apparatus 201 is actually worn on the body. When detecting no target within the certain distance from the electronic apparatus 201, the processor 220 can determine that the electronic apparatus 201 is not worn on the body. Although detecting the target within the certain distance from the electronic apparatus 201, when another pre-designated sensor (e.g., a heart rate sensor) does not detect sensor data, the processor 220 can determine that the electronic apparatus 201 is not worn on the body.

When detecting the context where the user wears the electronic apparatus 201, the processor 220 can activate the second sensor. For example, the processor 220 can determine the wearing state based on the at least one first sensor or the at least one second sensor. When detecting a motion of the electronic apparatus 201 corresponding to a direction or a distance to the body based on the first sensor, the processor 220 can determine that the electronic apparatus 201 is worn on the body. When detecting proximity or bio information of the body based on the second sensor, the processor 220 can determine that the electronic apparatus 201 is worn on the body.

Upon determining the wearing state of the electronic apparatus 201, the processor 220 can determine whether the electronic apparatus 201 is abnormally worn on the body of a user based on the detected motion of the electronic apparatus 201. For example, the abnormal wearing state of the electronic apparatus 201 can include a state where the electronic apparatus 201 is not normally fitted in the body, a state where the electronic apparatus 201 is not worn on the body in a normal direction or orientation, and the like. When sensor data of the sensor for at least one axis obtained in the wearing state corresponds to a preset reference range, the processor 220 can determine the normal wearing of the electronic apparatus 201. The preset reference range can be related to a motion range of the electronic apparatus 201 normally worn. When the sensor data of the sensor for the at least one axis obtained in the wearing state does not correspond to the preset reference range, the processor 220 can determining the abnormal wearing state of the electronic apparatus 201.

The processor 220 can determine a preset range of sensor information based on activity of the electronic apparatus 201. For example, the processor 220 can predict the activity of the electronic apparatus 201 or the user of the electronic apparatus 201, compare the reference range of the sensing information corresponding to the predicted activity with the sensor data obtained in the current wearing state, and thus determine whether the electronic apparatus 201 is normally worn. The processor 220 can determine a motion pattern (e.g., a step count, a stride length, a speed) of the electronic apparatus 201 using the sensor data obtained by at least one sensor of the electronic apparatus 201 in the wearing state, and determine the activity of the electronic apparatus 201 or the user wearing the electronic apparatus 201 based on the determined pattern. The processor 220 can determine at least one activity state from walking, running, cycling, exercising, and driving. The processor 220 may determine a facial movement (e.g., nodding, shaking) of the user wearing the electronic apparatus 201. The processor 220 can predict the activity of the electronic apparatus 201 based on a previous activity pattern and a current activity of the electronic apparatus 201. When the user switches from a previous activity pattern (e.g., walking) to a current activity (e.g., a stationary activity), the processor 220 can determine that the user of the electronic apparatus 201 is currently standing. The processor 220 may predict the user's activity (e.g., turning his/her head while standing) based on the current activity (e.g., standing) of the electronic apparatus 201.

When determining the abnormal wearing of the electronic apparatus 201, the processor 220 can control the electronic apparatus 201 based on the abnormal wearing state. For example, the processor 220 can output guide information so that the electronic apparatus 201 is normally worn. When the electronic apparatus 201 is not normally fitted into the body, the processor 220 can output the guide information of a sound or vibrations so that the electronic apparatus 201 is fitted in the body more tightly. When the electronic apparatus 201 is not worn in the normal direction, the processor 220 can output the guide information to notify the user to wear the electronic apparatus 201 in the normal direction.

When the electronic apparatus 201 communicates with another electronic apparatus, the processor 220 can output the guide information through the another electronic apparatus. For example, the processor 220 can process the output of auditory, haptic, or visual guide information through an output device (e.g., a display, a speaker, a motor) of the another electronic apparatus.

When the electronic apparatus 201 is not worn in the normal direction, the processor 220 can change its setting based on the abnormal direction.

The memory 230 can include a volatile and/or non-volatile memory. The memory 230 can store commands or data relating to other components of the electronic apparatus 201. According to an embodiment of the present disclosure, the memory 230 can store information used to determine the normal wearing of the electronic apparatus 201. For example, the information used to determine the normal wearing state can be related to the sensor data detected in the normal wearing state of the electronic apparatus 201.

The memory 230 can store software and/or a program 240. The program 240 can include, for example, a kernel 241, middleware 243, an application programming interface (API) 245, and/or an application program (or "application") 247. At least part of the kernel 241, the middleware 243, or the API 245 can be referred to as an operating system (OS).

The kernel 241 can control or manage system resources such as the bus 210, the processor 220, or the memory 230, used for performing operations or functions implemented by the other programs including the middleware 243, the API 245, or the application program 247. Additionally, the kernel 241 can provide an interface for controlling or managing system resources by accessing an individual component of the electronic device 201 from the middleware 243, the API 245, or the application program 247.

The middleware 243 can serve an intermediary role for allowing the API 245 or the application program 247 to communicate with the kernel 241. Additionally, the middleware 243 can process one or more job requests received from the application program 247, based on their priority. For example, the middleware 243 can assign a priority for using a system resource (e.g., the bus 210, the processor 220, or the memory 230) of the electronic device 201 to at least one of the application programs 247, and process the one or more job requests.

The API 245 is an interface through which the application 247 controls a function provided from the kernel 241 or the middleware 243, and can include at least one an instruction for file control, window control, image processing, or character control. The input/output interface 250 can deliver commands or data inputted from a user or another external device to other component(s) of the electronic device 201, or output commands or data inputted from the other component(s) of the electronic device 201 to the user or another external device.

The display 260 can include a liquid crystal display (LCD), a light emitting diode (LED) display, an organic light emitting diode (OLED) display, a microelectromechanical systems (MEMS) display, or an electronic paper display. The display 260 can display contents such as texts, images, videos, icons, and/or symbols, to the user. The display 260 can include a touch screen and receive touch, gesture, proximity, or hovering inputs by using an electronic pen or a user's body part. The communication interface 270 can set a communication between the electronic device 201 and an external device (e.g., a first external electronic device 202, a second external electronic device 204, or a server 206). The communication interface 270 can communicate with the external device over a network 262 through wireless communication or wired communication.

The wireless communication, for example, can include cellular communication using at least one of long term evolution (LTE), LTE-advanced (LTE-A), code division multiple access (CDMA), wideband CDMA (WCDMA), universal mobile telecommunications system (UMTS), wireless broadband (WiBro), or global system for mobile communications (GSM). The wireless communication can include at least one of wireless fidelity (WiFi), bluetooth, bluetooth low energy (BLE), Zigbee, near field communication (NFC), magnetic secure transmission, radio frequency (RF), and body area network (BAN). The wireless communication can include GNSS. The GNSS can include, for example, global positioning system (GPS), global navigation satellite system (GLONASS), Beidou navigation satellite system (Beidou), or Galileo (the European global satellite-based navigation system). Hereafter, the term "GPS" can be used interchangeably with the term "GNSS". The wired communication, for example, can include at least one of universal serial bus (USB), high definition multimedia interface (HDMI), recommended standard 232 (RS-232), power line communications, and plain old telephone service (POTS). The network 262 can include a telecommunications network at least one of computer network (e.g., LAN or WAN), internet, and telephone network.

The first and second external electronic devices 202 and 204 can be a same type or a different type of electronic device as that of the electronic device 201. All or part of operations executed in the electronic device 201 can be executed by another electronic device or a plurality of electronic devices (e.g., the first and second external electronic devices 202 and 204, or the server 206). To perform a function or service automatically or by request, instead of performing the function or the service by the electronic device 201, the electronic device 201 can request at least part of a function relating thereto from another device (e.g., the first and second external electronic devices 202 and 204, or the server 206). The other electronic device (e.g., the first and second external electronic devices 202 and 204, or the server 206) can perform the requested function or an additional function and send its result to the electronic device 201. The electronic device 201 can provide the requested function or service by processing the received result. In doing so, cloud computing, distributed computing, or client-server computing techniques can be used.

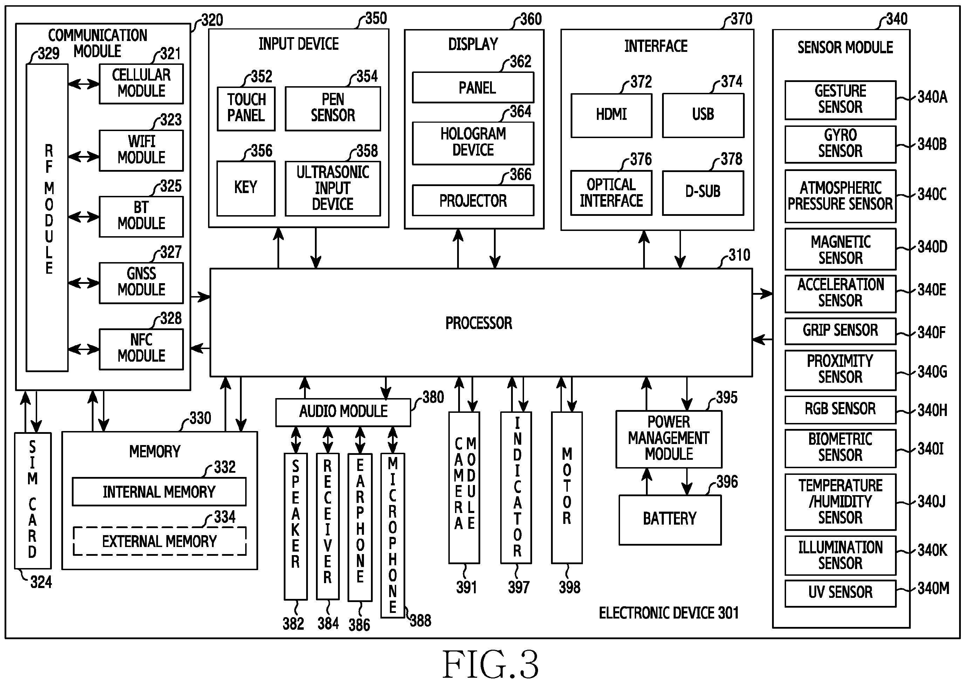

FIG. 3 is a block diagram of an electronic apparatus according to embodiments of the present disclosure;

The electronic device 301 can include all or part of the above-described electronic device 201 of FIG. 2. The electronic device 301 includes one or more processors (e.g., an AP) 310, a communication module 320, a subscriber identification module (SIM) 324, a memory 330, a sensor module 340, an input device 350, a display 360, an interface 370, an audio module 380, a camera module 391, a power management module 395, a battery 396, an indicator 397, and a motor 398.

The processor 310 can control a plurality of hardware or software components connected to the processor 310, and also can perform various data processing and operations by executing an OS or an application program. The processor 310 can be implemented with a system on chip (SoC). The processor 310 can further include a graphic processing unit (GPU) and/or an image signal processor. The processor 310 may include at least some (e.g., a cellular module 321) of the components shown in FIG. 2. The processor 310 can load commands or data received from at least one of the other components (e.g., a nonvolatile memory) onto a volatile memory, process the command or data, and store data in the nonvolatile memory.

The communication module 320 can have the same or similar configuration to the communication interface 170 of FIG. 1. The communication module 320 can include the cellular module 321, a WiFi module 323, a bluetooth (BT) module 325, a GNSS module 327, an NFC module 328, and an RF module 329. The cellular module 321 can provide voice call, video call, short message service (SMS), or Internet service through a communication network. The cellular module 321 can identify and authenticate the electronic device 301 in a communication network by using the SIM (e.g., a SIM card) 324.

The cellular module 321 can perform at least part of a function that the processor 310 provides. The cellular module 321 can further include a communication processor (CP). Two or more of the cellular module 321, the WiFi module 323, the BT module 325, the GNSS module 327, and the NFC module 328 can be included in one integrated circuit (IC) or an IC package. The RF module 329 can transmit/receive a communication signal (e.g., an RF signal). The RF module 329, for example, can include a transceiver, a power amp module (PAM), a frequency filter, a low noise amplifier (LNA), or an antenna. According to another embodiment, at least one of the cellular module 321, the WiFi module 323, the BT module 325, the GNSS module 327, and the NFC module 328 can transmit/receive an RF signal through an additional RF module. The SIM 324 can include a card having a SIM or an embedded SIM, and also can contain unique identification information, an integrated circuit card identifier (ICCID), or subscriber information such as an international mobile subscriber identity (IMSI).

The memory 330 and the memory 230 can include at least one of an internal memory 332 and an external memory 334. The internal memory 332 can include at least one of a volatile memory (e.g., dynamic RAM (DRAM), static RAM (SRAM), or synchronous dynamic RAM (SDRAM)), and a non-volatile memory (e.g., one time programmable ROM (OTPROM), programmable ROM (PROM), erasable and programmable ROM (EPROM), electrically erasable and programmable ROM (EEPROM), mask ROM, flash ROM, flash memory, hard drive, and solid state drive (SSD)). The external memory 334 can include flash drive compact flash (CF), secure digital (SD), micro SD, mini SD, extreme digital (xD), multi-media card (MMC), or memory stick. The external memory 334 can be functionally or physically connected to the electronic device 301 through various interfaces.

The sensor module 340 can measure physical quantities or detect an operating state of the electronic device 301, and thus convert the measured or detected information into electrical signals. The sensor module 340 can include at least one of a gesture sensor 340A, a gyro sensor 340B, an atmospheric pressure sensor 340C, a magnetic sensor 340D, an acceleration sensor 340E, a grip sensor 340F, a proximity sensor 340G, a color sensor 340H, a red, green, blue (RGB) sensor, a bio sensor 340I, a temperature/humidity sensor 340J, an illumination sensor 340K, and an ultra violet (UV) sensor 340M. The sensor module 340 can include an E-nose sensor, an electromyography (EMG) sensor, an electroencephalogram (EEG) sensor, an electrocardiogram (ECG) sensor, an infrared (IR) sensor, an iris sensor, and/or a fingerprint sensor. The sensor module 340 can further include a control circuit for controlling at least one sensor therein. The electronic device, as part of the processor 310 or individually, can further include a processor configured to control the sensor module 340 and thus control the sensor module 340 while the processor 310 is sleeping.

The input device 350 can include at least one of a touch panel 352, a digital pen sensor 354, a key 356, and an ultrasonic input device 358. The touch panel 352 can use at least one of capacitive, resistive, infrared, and ultrasonic methods. Additionally, the touch panel 352 can further include a control circuit. The touch panel 352 can further include a tactile layer to provide a tactile response to a user. The digital pen sensor 354 can include part of a touch panel or a sheet for recognition. The key 356 can include a physical button, a touch key, an optical key, or a keypad. The ultrasonic input device 358 can detect ultrasonic waves from an input means through a microphone 388 and check data corresponding to the detected ultrasonic waves.

The display 360 or the display 260 can include at least one of a panel 362, a hologram device 364, a projector 366, and/or a control circuit for controlling them. The panel 362 can be implemented to be flexible, transparent, or wearable, for example. The panel 362 and the touch panel 352 can be configured with one or more modules. The panel 362 can include a pressure sensor (or a force sensor) for measuring a pressure of the user's touch. The pressure sensor can be integrated with the touch panel 352, or include one or more sensors separately from the touch panel 352. The hologram device 264 can show three-dimensional images in the air by using the interference of light. The projector 366 can display an image by projecting light on a screen. The screen can be placed inside or outside the electronic device 301. The interface 370 can include an HDMI 372, a USB 374, an optical interface 376, or a D-subminiature (D-sub) 378. The interface 370 can be included in the communication interface 270 of FIG. 2. Additionally or alternately, the interface 370 can include a mobile high-definition link (MHL) interface, a SD card/MMC interface, or an Infrared Data Association (IrDA) standard interface.

The audio module 380 can convert sounds into electrical signals and convert electrical signals into sounds. At least some components of the audio module 380 can be included in the input/output interface 250 of FIG. 2. The audio module 380 can process sound information inputted or outputted through a speaker 382, a receiver 384, an earphone 386, or the microphone 388. The camera module 391 can be a device for capturing still images and videos and can include one or more front and back image sensors, a lens, an image signal processor (ISP), or a flash such as an LED or a xenon lamp.

The power management module 395 can manage the power of the electronic device 301 and can include a power management IC (PMIC), a charger IC, or a battery gauge. The PMIC can have a wired and/or wireless charging method. The wireless charging method can include a magnetic resonance method, a magnetic induction method, or an electromagnetic method, and can further include an additional circuit for wirelessly charging a coil loop, a resonant circuit, or a rectifier circuit. The battery gauge can measure the remaining capacity of the battery 396, or a voltage, current, or temperature of the battery 396 during charging. The battery 396 can include a rechargeable battery and/or a solar battery.

The indicator 397 can display a specific state of the electronic device 301 or part thereof (e.g., the processor 310), such as a booting state, a message state, or a charging state. The motor 398 can convert electrical signals into mechanical vibration and generate a vibration or haptic effect. The electronic device 301 can include a mobile TV supporting device (e.g., a GPU) for processing media data according to standards such as digital multimedia broadcasting (DMB), digital video broadcasting (DVB), or mediaFlo™. Each of the above-described components of the electronic device 301 can be configured with at least one component and the name of a corresponding component can vary according to the kind of electronic device. According to an embodiment of the present disclosure, an electronic device 301 can be configured to include at least one of the above-described components, an additional component, or to not include some of the above-described components. Additionally, some of components in an electronic device are configured as one entity, so that functions of previous corresponding components are performed identically.

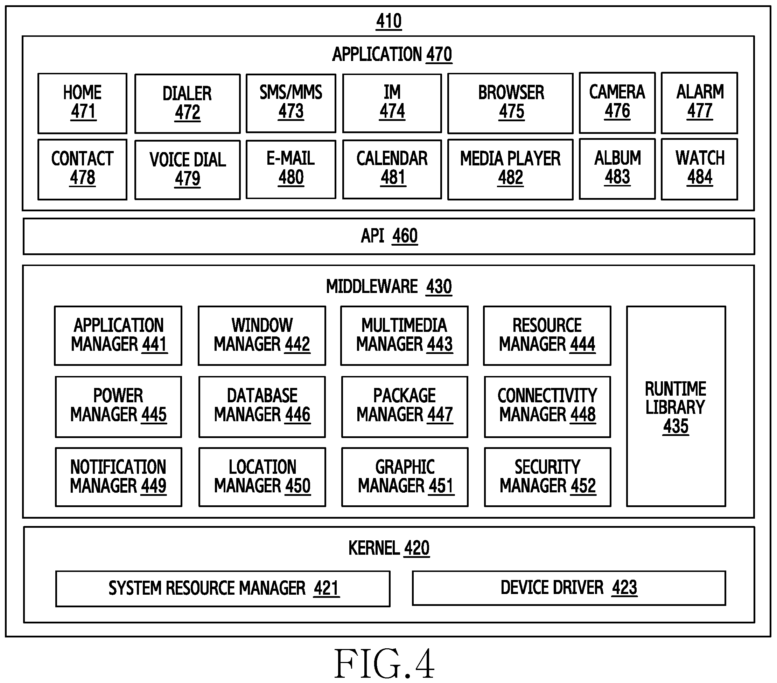

FIG. 4 is a block diagram of a program module according to an embodiment of the present disclosure. A program module 410 and/or the program 240 can include an OS for controlling a resource relating to an electronic device 201 and/or the application program 247 running on the OS. The OS can include, for example, Android™, iOS™, Windows™, Symbian™, Tizen™, or Bada™.

The program module 410 can include a kernel 420, a middleware 430, an API 460 and/or applications 470 as the application program 247. At least part of the program module 410 can be preloaded on an electronic device or can be downloaded from the electronic device 202, 104, or the server 206.

The kernel 420 includes at least one of a system resource manager 421 and/or a device driver 423. The system resource manager 421 can control, allocate, or retrieve a system resource. The system resource manager 421 can include a process management unit, a memory management unit, or a file system management unit. The device driver 423 can include a display driver, a camera driver, a bluetooth driver, a sharing memory driver, a USB driver, a keypad driver, a WiFi driver, an audio driver, or an inter-process communication (IPC) driver.

The middleware 430 can provide a function commonly required by the application 470, or can provide various functions to the application 470 through the API 460 in order to allow the application 470 to efficiently use a limited system resources inside the electronic device. The middleware 430 includes at least one of a runtime library 435, an application manager 441, a window manager 442, a multimedia manager 443, a resource manager 444, a power manager 445, a database manager 446, a package manager 447, a connectivity manager 448, a notification manager 449, a location manager 450, a graphic manager 451, and a security manager 452.

The runtime library 435 can include a library module used by a complier to add a new function through a programming language while the application 470 is running. The runtime library 435 can manage input/output, manage memory, or arithmetic function processing. The application manager 441 can manage the life cycle of the applications 470. The window manager 442 can manage a GUI resource used in a screen. The multimedia manager 443 can recognize a format for playing various media files and encode or decode a media file by using the codec in a corresponding format. The resource manager 444 can manage a source code of the applications 470 or a memory space. The power manager 445 can manage the capacity or power of the battery and provide power information for an operation of the electronic device. The power manager 445 can operate together with a basic input/output system (BIOS). The database manager 446 can create, search, or modify a database used in the applications 470. The package manager 447 can manage installation or updating of an application distributed in a package file format. The connectivity manger 448 can manage a wireless connection. The notification manager 449 can provide an event, such as incoming messages, appointments, and proximity alerts, to the user. The location manager 450 can manage location information of an electronic device. The graphic manager 451 can manage a graphic effect to be provided to the user or a user interface relating thereto. The security manager 452 can provide system security or user authentication.

The middleware 430 can include a telephony manager for managing a voice or video call function of the electronic device, or a middleware module for combining various functions of the above-described components. The middleware 430 can provide a module specialized for each type of OS. The middleware 430 can dynamically delete part of the existing components or add new components.

The API 460, as a set of API programming functions, can be provided as another configuration according to the OS. For example, Android™ or iSO™ can provide one API set for each platform, and Tizen™ can provide two or more API sets for each platform.

The applications 470 can include a home application 471, a dialer application 472, an SMS/multimedia messaging system (MMS) application 473, an instant message (IM) application 474, a browser application 475, a camera application 476, an alarm application 477, a contact application 478, a voice dial application 479, an e-mail application 480, a calendar application 481, a media player application 482, an album application 483, a clock application 484, a health care application for measuring an exercise amount or blood sugar level, or an environmental information application for measuring air pressure, humidity, or temperature information. The applications 470 can include an information exchange application for supporting information exchange between the electronic device and an external electronic device. The information exchange application can include a notification relay application for relaying specific information to the external device or a device management application for managing the external electronic device. The notification relay application can relay notification information from another application of the electronic device to an external electronic device, or receive and forward notification information from an external electronic device to the user. The device management application can install, delete, or update a function to turn-on/turn off the external electronic device (or some components), to display a brightness (or resolution) adjustment of an external electronic device communicating with the electronic device, or to operate an application in the external electronic device. The applications 470 can include a specified application (e.g., a health care application of a mobile medical device) according to a property of the external electronic device. The applications 470 can include an application received from an external electronic device. At least part of the program module 310 can be implemented (e.g., executed) with software, firmware, hardware (e.g., the processor 210), or a combination of at least two of them, and include a module, a program, a routine, a set of instructions, or a process for executing one or more functions.

The term "module" used in the present disclosure includes a unit consisting of hardware, software, or firmware, and may be used interchangeably with a term such as "unit", "logic", "logical block", "component", "circuit", and the like. The "module" may be an integrally constructed component or a minimum unit or one part thereof for performing one or more functions. The "module" may be mechanically or electrically implemented, and may include, for example, an application-specific integrated circuit (ASIC) chip, a field-programmable gate arrays (FPGAs), or a programmable-logic device, which is known or developed to perform certain operations. At least one part of an apparatus (e.g., modules or functions thereof) or methods (e.g., operations) according to embodiments of the present disclosure may be implemented with an instruction stored in a computer-readable storage media (e.g., the memory 230). If the instruction is executed by one or more processors (e.g., the processor 220), the one or more processors may perform a function corresponding to the instruction. The computer-readable storage media may include a hard disk, a floppy disk, magnetic media (e.g., a magnetic tape), optical media (e.g., a compact disc-ROM (CD-ROM), a digital versatile disc (DVD), magnetic-optic media (e.g., a floptical disk)), an internal memory, or the like. The instruction may include a code created by a compiler or a code executable by an interpreter. A module or program module may further include at least one or more of the aforementioned constituent elements, or the module or program module may omit some constituent elements. Operations carried out by a module, a program module or another constituent element may be executed in a sequential, parallel, repeated or heuristic manner, or at least some operations may be executed in different order, may be omitted, or may be added.

An electronic apparatus according to the present disclosure includes a motion sensor, an optical sensor, a memory, and a processor. The processor can determine whether the electronic apparatus is worn on a body using at least one of the motion sensor and the optical sensor, when determining the wearing, obtain sensor data according to a motion of the electronic apparatus using at least one of the motion sensor and the optical sensor, compare the obtained data with at least one reference information stored in the memory, determine whether the electronic apparatus is normally worn based on the comparison, and perform at least one operation based on the determination.

The processor can determine an activity state of the electronic apparatus using at least one of the motion sensor and the optical sensor, and obtain reference information corresponding to the determined activity state.

When determining the normal wearing state, the processor can detect detachment of the electronic apparatus using at least one of the motion sensor and the optical sensor, and output guide information notifying the detachment of the electronic apparatus.

When determining an abnormal wearing state, the processor can output guide information notifying the abnormal wearing state, or the processor can change a setting of the electronic apparatus.

The electronic apparatus can further include a communication unit. The processor can communicate with another electronic apparatus through communication unit, and receive normal or abnormal wearing information from the another electronic apparatus.

The processor can determine whether the wearing of the electronic apparatus is normal based on a wearing direction of the electronic apparatus.

The processor can determine whether the electronic apparatus is normally worn based on acceleration information for at least one axis of the motion sensor.

The electronic apparatus can include an earbud.

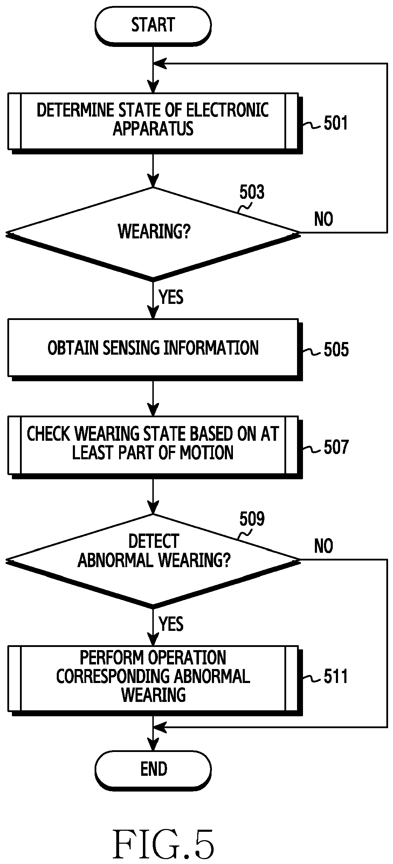

FIG. 5 is a flowchart of a method for determining a normal wearing state in an electronic apparatus 201 according to embodiments of the present disclosure.

In step 501, the electronic apparatus 201 can determine its movement or proximity to a target based on at least one sensor. For example, the electronic apparatus 201 can determine its state using at least one of an inertial sensor, an acceleration sensor, a geomagnetic sensor, a gyro sensor, a photoelectric sensor, an infrared sensor, a bio sensor, an electromagnetic sensor, a grip sensor, and an EMG sensor.

In step 503, the electronic apparatus 201 can determine whether the state of the worn electronic apparatus 201 is detected. When detecting a movement (or a movement change) of the electronic apparatus 201 corresponding to a certain direction or a certain speed based on the inertial sensor, the electronic apparatus 201 can determine available wearing state detected. When detecting the available wearing state and detecting a target within a certain distance from the electronic apparatus 201 based on the photoelectric sensor and/or the electromagnetic sensor, the electronic apparatus 210 can determine the actual wearing state on the body.

When determining that the apparatus is not being worn, the electronic apparatus 201 can determine whether it is worn on the body and perform step 501 or step 503.

When determining the wearing state, the electronic apparatus 201 can obtain sensor data (or sensing information) from the at least one sensor in step 505. The electronic apparatus 201 which is worn on the body can obtain the sensor data from its sensor.

In step 507, the electronic apparatus 201 can determine whether it is normally worn on or not based on at least part of the obtained sensor data. In the normal wearing state, the electronic apparatus 201 is fitted in the body in a preset wearing direction. The electronic apparatus 201 is designed to wear on (or fit in) a left (or right) ear of the body of a user and can be worn on the left (or right) ear of the user. In the abnormal wearing state, the electronic apparatus 201 is not worn in the preset wearing direction. In the normal wearing state, the electronic apparatus 201 is tightly attached to the body in the user's movement. In the abnormal wearing state, the electronic apparatus 201 is not tightly attached to the user's body but also not detached from the body. The electronic apparatus 201 can obtain the user's activity state based on the sensor data obtained in the wearing state. The electronic apparatus 201 can determine normal or abnormal wearing by comparing a reference range corresponding to the user's activity state among reference ranges stored in the memory 230, with the motion of the electronic apparatus 201. For example, the reference range can indicate a range and a pattern of the sensor data detected by the electronic apparatus 201 normally worn, with respect to at least one axis of the sensor. When the reference range for at least one axis (e.g., X axis) of the sensor (e.g., the acceleration sensor, the gyro sensor, etc.) of the electronic apparatus 201 is a first range (e.g., 0 ∼ +25 m/s2) and the electronic apparatus 201 obtains sensor data within the first range from at least one sensor, the electronic apparatus 201 can determine the normal wearing state. When obtaining sensor data in a second range out of the reference range from at least one sensor and the electronic apparatus 201 designed for the left ear of the body is worn on, or fitted in, the right ear of the user, the electronic apparatus 201 can determine the abnormal wearing state.

When measuring bio information or determining at least part of the body based on the sensor data obtained in the wearing state, the electronic apparatus 201 can determine the normal wearing state. When not measuring the bio information or not determining at least part of the body based on the sensor data obtained in the wearing state, the electronic apparatus 201 can determine that it is mounted in another space (e.g., a portable case) other than the body.

In step 509, the electronic apparatus 201 can determine whether abnormal wearing step is detected.

When detecting the normal wearing state, the electronic apparatus 201 can control its operation in response to detection of the normal wearing state.

When detecting the abnormal wearing state, the electronic apparatus 201 can perform an operation corresponding to the abnormal wearing state in step 511. The operation corresponding to the abnormal wearing state can output guide information to guide the normal wearing or to notify the user of abnormal wearing. For example, the operation corresponding to the abnormal wearing state can change a setting of the electronic apparatus 201 based on a current wearing direction. When the electronic apparatus 201 designed for the left ear of the user's body is worn on, fitted in, the right ear, the electronic apparatus 201 can change its setting and operate as an electronic apparatus designed for the right ear.

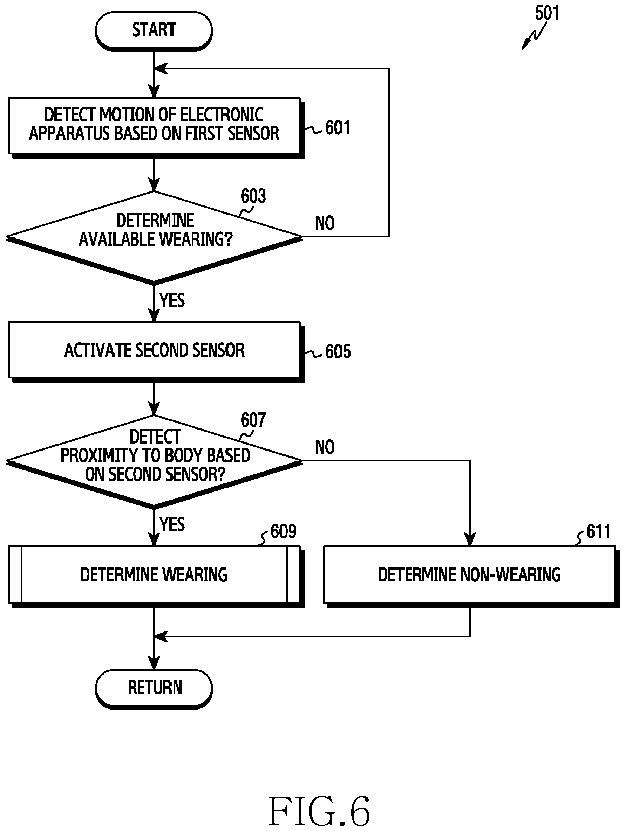

FIG. 6 is a flowchart of a method for determining a wearing state of an electronic apparatus 201 according to step 501 of FIG. 5.

Referring to FIG. 6, the electronic apparatus 201 can determine its state based on a first sensor and a second sensor. For example, the first sensor can consume relatively lower power than the second sensor. The first sensor can include at least one inertial sensor, an acceleration sensor, a geomagnetic sensor, or a gyro sensor for determining a movement of the electronic apparatus 201. The second sensor can include at least one inertial sensor, an infrared sensor, a bio sensor and/or an electromagnetic sensor, a grip sensor, or an EMG sensor for determining proximity to a target.

In step 601, the electronic apparatus 201 can detect its movement based on the first sensor. The electronic apparatus 201 can detect a movement change for at least one axis of the sensor.

In step 603, the electronic apparatus 201 can determine whether available wearing is detected based on the detected movement. For example, when detecting a movement, or a movement change, corresponding to a certain direction or a certain speed (e.g., a movement toward the body), the electronic apparatus 201 can determine the available wearing detected. When detecting a movement, or a movement change, not corresponding to the certain direction or the certain speed, the electronic apparatus 201 can determine that the available wearing not detected.

When not detecting the available wearing, the electronic apparatus 201 can perform step 601 and detect its movement. In step 605, when detecting the available wearing, the electronic apparatus 201 can activate the second sensor.

In step 607, the electronic apparatus 201 can determine whether proximity to the body of a user is detected based on the second sensor. For example, when detecting a target such as the body, within a certain distance from the electronic apparatus 201 or obtaining bio information, the electronic apparatus 201 can determine the proximity to the body. When not detecting the target within the certain distance from the electronic apparatus 201 or not obtaining the bio information, the electronic apparatus 201 can determine no proximity to the body.

In step 611, when not determining the body proximity while the available wearing is detected, the electronic apparatus 201 can determine that it is not being worn.

In step 609, when determining the body proximity while the available wearing is detected, the electronic apparatus 201 can determine the wearing state.

According to embodiments of the present disclosure, the electronic apparatus 201 can process the activated second sensor in association with other function. For example, the electronic apparatus 201 can output information indicating that the second sensor is activated. When at least one function in association with the second sensor is selected, the electronic apparatus 201 can execute the selected function (e.g., health care) based on sensor data obtained by the second sensor.

The electronic apparatus 201 may omit at least one of the steps of FIG. 6. For example, the electronic apparatus 201 can determine the state when both the first sensor and the second sensor are activated. In this case, the electronic apparatus 201 can omit step 605.

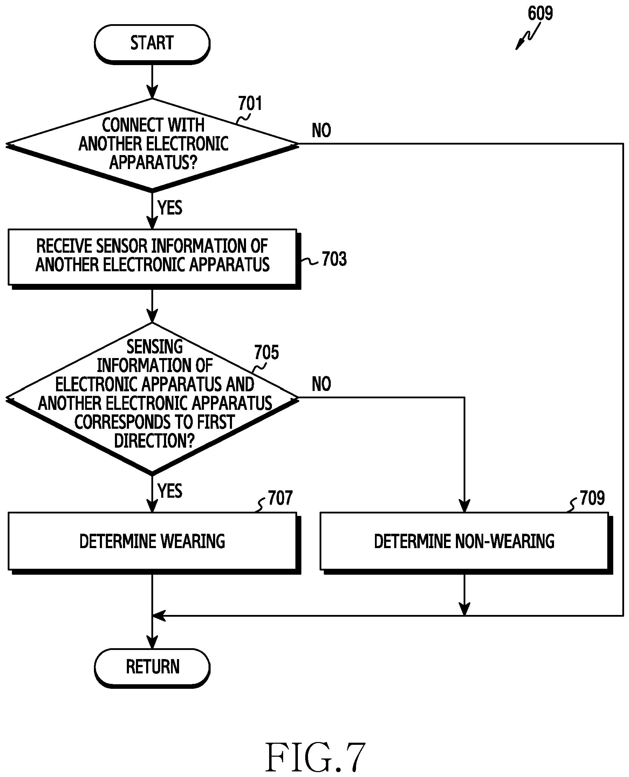

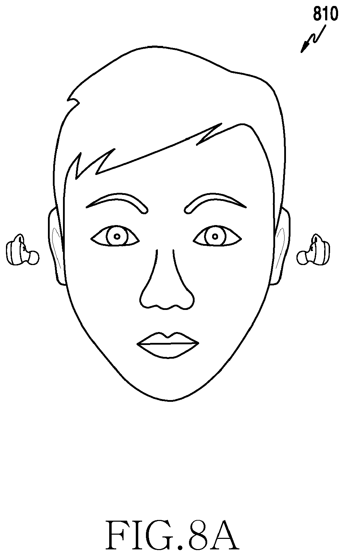

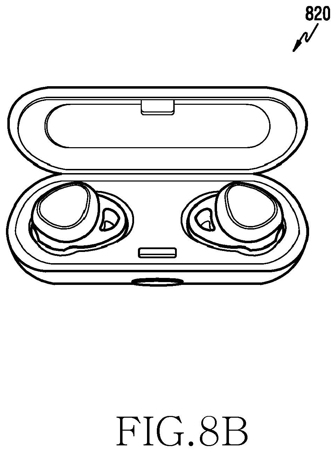

FIG. 7 is a flowchart of a method for determining wearing of an electronic apparatus 201. FIGS. 8A and 8B are diagrams for determining wearing. According to various embodiments of the present disclosure, determining the wearing state can include detailed operations of step 609 of FIG. 6.

Referring to FIG. 7, the electronic apparatus 201 may determine the wearing state based on a first sensor, a second sensor, and sensor data of another electronic apparatus. For example, even when the electronic apparatus 201 is put in a portable case, a pocket, or a bag, the electronic apparatus 201 can obtain sensor data satisfying a condition through the first sensor and the second sensor and thus determine the wearing state. In this regard, the electronic apparatus 201 can determine the wearing state using sensor data of another electronic apparatus.

In step 701, the electronic apparatus 201 can determine whether another electronic apparatus is connected. For example, the electronic apparatus 201 and the another electronic apparatus can organize a set. When the electronic apparatus 201 is a first electronic apparatus of an ear-wearable type designed for the left ear, the another electronic apparatus can be a second electronic apparatus of the ear-wearable type designed for the right ear. The electronic apparatus 201 can determine whether the another electronic apparatus is connected based on short-range communication such as bluetooth (BT), ANT+, and wireless fidelity (Wi-Fi).

Upon determining a connection with the another electronic apparatus, the electronic apparatus 201 can receive sensor data from the connected another electronic apparatus (e.g., the second electronic apparatus) in step 703. For example, the electronic apparatus 201 can obtain the sensor data from at least one sensor of the another electronic apparatus.

In step 705, the electronic apparatus 201 can determine whether the electronic apparatus 201 and the another electronic apparatus obtain the sensor data corresponding to a first posture. The electronic apparatus 201 can obtain the posture of the another electronic apparatus by comparing a reference range of the another electronic apparatus with the sensor data received from the another electronic apparatus. For example, in the first posture, at least one axis (e.g., Z axis) of the sensor of the electronic apparatus 201 forms a depth direction (thickness direction) of the main body, another axis (e.g., X axis) of the sensor forms a width direction of the main body, and the other axis (e.g., Y axis) of the sensor forms a height direction of the main body. In a second posture, the axis of at least one direction of the electronic apparatus 201 does not match the axis of the first posture.

When obtaining the sensor data corresponding to the first posture, the electronic apparatus 201 can determine whether it is being worn in step 707. For example, when the electronic apparatus 201 and the another electronic apparatus maintain a first posture 810 of FIG. 8A, the electronic apparatus 201 can determine the wearing.

When determining no sensor data corresponding to the first posture, the electronic apparatus 201 can determine non-wearing in step 709. For example, when the electronic apparatus 201 and the another electronic apparatus maintain a second posture 820 of FIG. 8B, the electronic apparatus 201 can determine that the electronic apparatus 201 is located in a space such as a portable case, rather than the body.

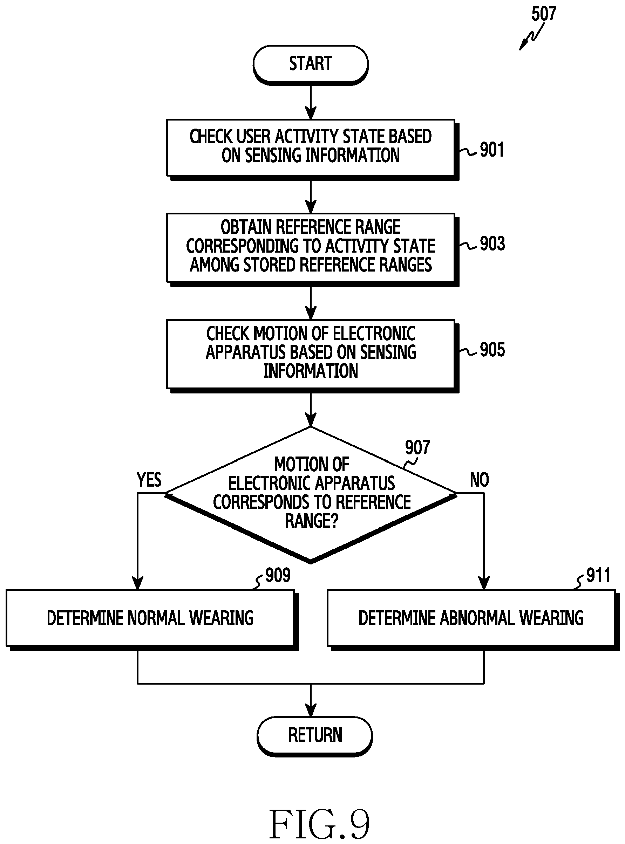

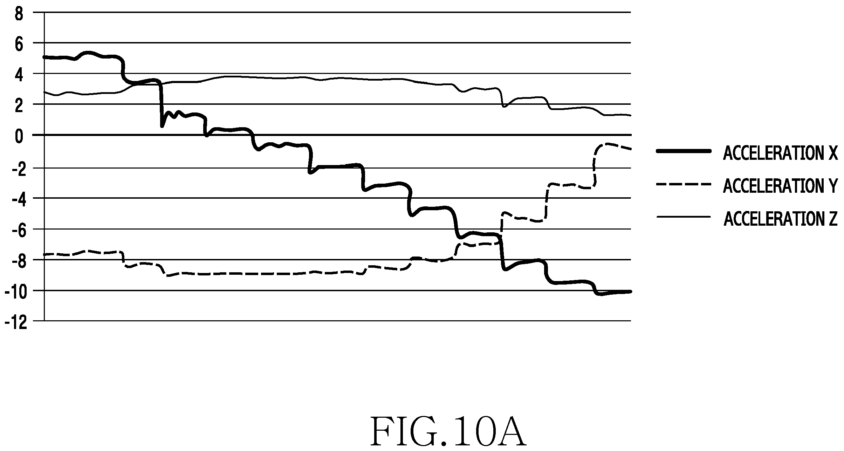

FIG. 9 is a flowchart of a method for determining a wearing state of an electronic apparatus 201. FIGS. 10A through 10D are graphs of sensor data in determining a wearing state according to embodiments of the present disclosure. Determining the wearing state can include detailed operations of step 507 of FIG. 5. Referring to FIG. 9, in step 901, the electronic apparatus 201 can obtain a user's activity state based on sensor data according to a movement change for at least one axis measured in the wearing state.

When not detecting an acceleration change with respect to the Z axis of the sensor corresponding to the depth (thickness) of the main body of the electronic apparatus 201, the X axis of the sensor corresponding to the width of the main body, and the Y axis of the sensor corresponding to the height of the main body, then the electronic apparatus 201 can determine a static user activity.

When detecting an acceleration change of a certain level for the Z axis and the Y axis of the sensor of the electronic apparatus 201 and detecting an acceleration change below a certain level for the X axis of the sensor of the electronic apparatus 201, the electronic apparatus 201 can determine that the static user activity is a head nod.

When detecting an acceleration change of a certain level for the Y axis and the X axis of the sensor of the electronic apparatus 201 and detecting an acceleration change below the certain level for the Z axis of the sensor of the electronic apparatus 201 as shown in FIG. 10A, the electronic apparatus 201 can determine that the static user activity is a turn of a user's head to the left or to the right.

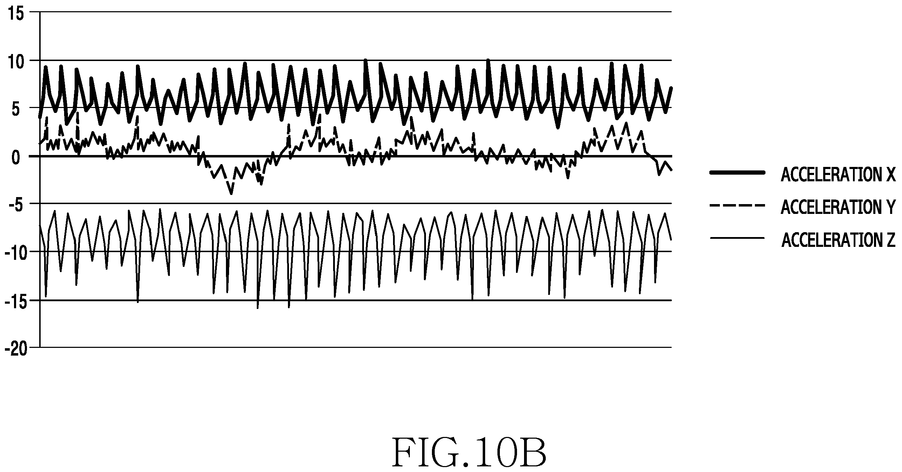

When detecting an acceleration change in a certain pattern for the Z axis, the X axis, and the Y axis of the sensor of the electronic apparatus 201 as shown in FIG. 10B, the electronic apparatus 201 can determine a dynamic user activity. Based on a width or a size of the pattern, the electronic apparatus 201 may determine walking, running, cycling, exercising, or driving.

The electronic apparatus 201 can determine the user's activity based on activity determination history. For example, when the static activity continues, the electronic apparatus 201 can determine that the user is standing, sitting, or lying. When the dynamic state switches to the static state, the electronic apparatus 201 may determine that the user moves his/her head in the stationary state.

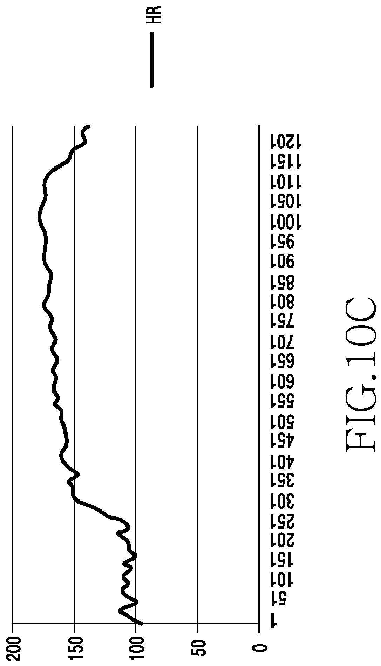

The electronic apparatus 201 can determine the user's activity based on a change of bio information obtained by the sensor. For example, when the heart rate increases to a certain level as shown in FIG. 10C, the electronic apparatus 201 can determine the dynamic user activity.

In step 903, the electronic apparatus 201 can obtain at least one reference range corresponding to the activity state among reference ranges stored in the memory 230. For example, when detecting the static activity, the electronic apparatus 201 can obtain a first reference range or a pattern of sensor data detected by at least one axis of the sensor of the electronic apparatus 201 worn on the user who maintains the static activity. The first reference range can be a range or a pattern of the sensor data detected when the user nods his/her head in the stationary state.

When detecting the dynamic activity, the electronic apparatus 201 can obtain a second reference range. The second reference range can be a range or a pattern of the sensor data detected by at least one axis of the sensor of the electronic apparatus 201 worn on the user who maintains the dynamic activity. The second reference range can be a range or a pattern of sensor data detected when the user is walking.

In step 905, the electronic apparatus 201 can obtain its movement based on the sensor data.

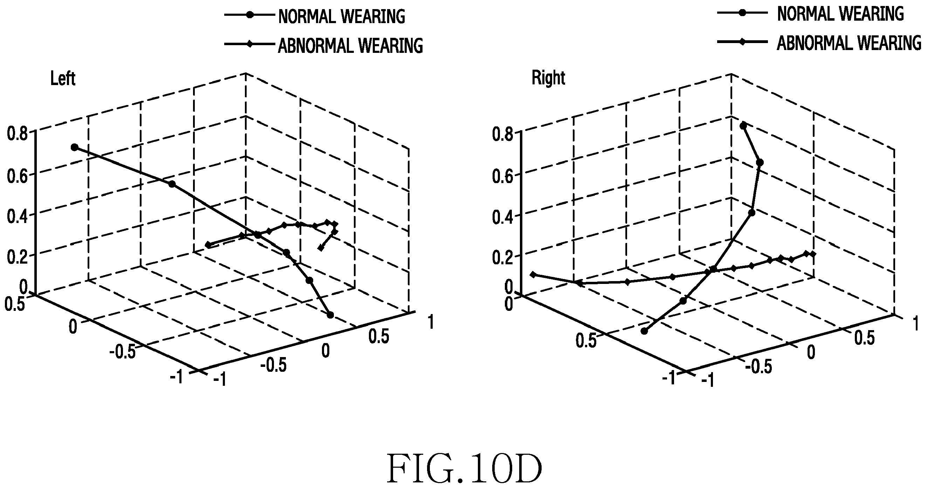

In step 907, the electronic apparatus 201 can determine whether the sensor data generated by the movement of the electronic apparatus 201 corresponds to the reference range of the activity. For example, as shown in FIG. 10D, The electronic apparatus 201 can store a trajectory (or range) of sensor data corresponding to the movement direction when the electronic apparatus 201 is normally worn on the left side or the right side of the body, and store a trajectory (or range) of sensor data corresponding to the movement direction in the abnormal wearing state. Also, the electronic apparatus 201 can determine whether the sensor data generated by its movement corresponds to the trajectory of the sensor data corresponding to the abnormal wearing state or the trajectory of the sensor data corresponding to the normal wearing state.

When the sensor data generated by the movement of the electronic apparatus 201 corresponds to the trajectory of the sensor data of the abnormal wearing state, the electronic apparatus 201 can determine abnormal wearing in step 911.

When the sensor data generated by the movement of the electronic apparatus 201 corresponds to the trajectory of the sensor data of the normal wearing state, the electronic apparatus 201 can determine normal wearing in step 909.

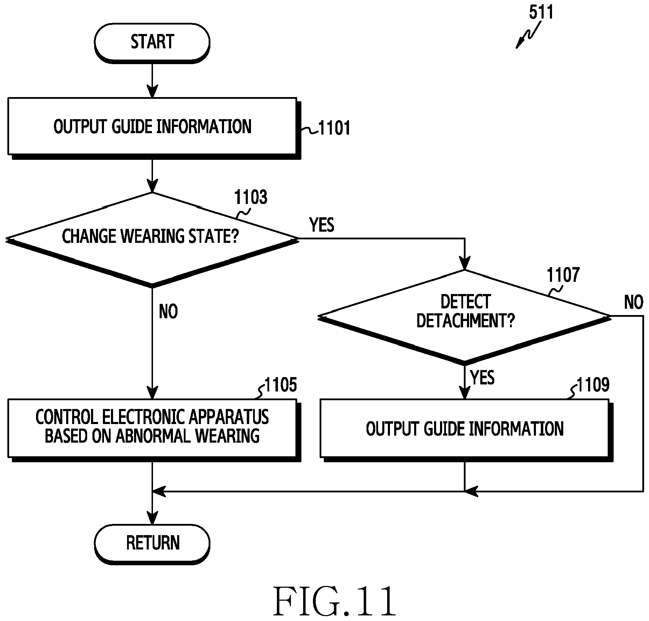

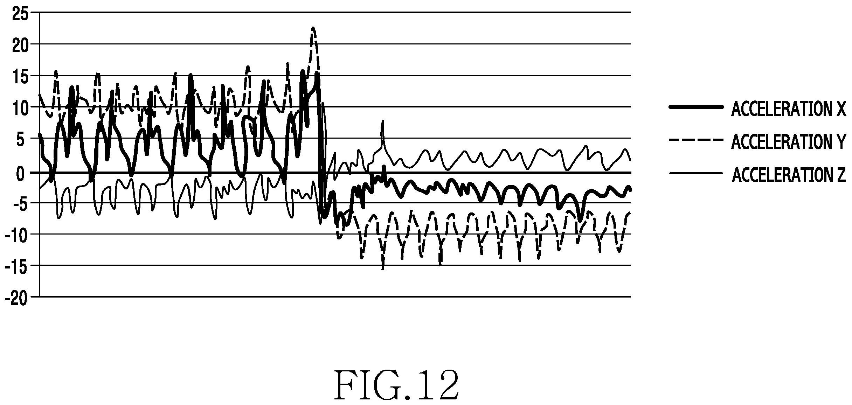

FIG. 11 is a flowchart of a method for abnormal wearing of an electronic apparatus 201. FIG. 12 is a graph of sensor data in relation to a wearing state change of the electronic apparatus 201. Operations for abnormal wearing of the electronic apparatus 201 can include detailed operations of step 511 of FIG. 5.

Referring to FIG. 11, when detecting the abnormal wearing state, the electronic apparatus 201 can output guide information indicating the abnormal wearing state in step 1101. For example, when the electronic apparatus 201 is not tightly worn on the body, the electronic apparatus 201 can output the guide information such as sound or vibrations so as to cause the user to wear the electronic apparatus 201 more tightly. When the electronic apparatus 201 is not worn in a normal direction, the electronic apparatus 201 can output the guide information to the user to wear it in the normal direction.

In step 1103, the electronic apparatus 201 can determine whether the wearing state changes to the normal wearing state within a certain time after the guide information is output. For example, as shown in FIG. 12, when obtaining sensor data (e.g., sensor data for the X axis and the Y axis) for at least one axis obtained in a first range (e.g., 0 ∼ +25 m/s2) based on a certain time in a second range (e.g., 0 ∼ -25 m/s2), the electronic apparatus 201 can determine the wearing state change.

When determining no wearing state change, the electronic apparatus 201 can control its operation based on the abnormal wearing state in step 1105. For example, the electronic apparatus 201 can change its setting based on a current wearing direction.

The electronic apparatus 201 can be designed for the left ear to output a sound corresponding to a first level. When this electronic apparatus 201 is worn on the right ear, the electronic apparatus 201 can change its volume to correspond to a volume of another electronic apparatus (e.g., the second electronic apparatus) designed for the right ear.

A key button of the electronic apparatus 201 designed for the left ear can generate a signal for controlling a first operation. When this electronic apparatus 201 is worn on the left ear, the electronic apparatus 201 can change its setting to generate a control signal of a key button of another electronic apparatus (e.g., the second electronic apparatus).

The electronic apparatus 201 designed for the left ear can be configured to output a sound corresponding to a first component. When this electronic apparatus 201 is worn on the right ear, the electronic apparatus 201 can change its setting to output a sound of a second component.

When determining the wearing state change, the electronic apparatus 201 can determine whether it is attached or detached in step 1107. For example, the electronic apparatus 201 can determine the detachment by detecting a distance change from at least part of the user's body. When the electronic apparatus 201 is spaced apart from at least part of the body over a certain distance, the electronic apparatus 201 can determine detachment of the electronic apparatus 201 from the body.

Upon determining the detachment, the electronic apparatus 201 can output guide information notifying the detachment in step 1109. The electronic apparatus 201 can change a guide information output type based on a distance change between the electronic apparatus and at least part of the body. For example, when detaching from at least part of the body at a preset distance, the electronic apparatus 201 can output guide information of a first basis. When detaching from at least part of the body over the preset distance, the electronic apparatus 201 can output guide information of a second basis.

According to embodiments of the present disclosure, a method for operating an electronic apparatus includes determining whether the electronic apparatus is worn on a user's body using at least one of a motion sensor and an optical sensor, obtaining sensor data according to a motion of the electronic apparatus using at least one of the motion sensor and the optical sensor when determining whether the electronic apparatus is worn on the user's body, comparing the obtained data with at least one reference information stored, determining whether the electronic apparatus is normally worn based on the comparison, and performing at least one operation based on the determination.

Comparing the obtained data with the at least one reference information stored can include determining an activity state of the electronic apparatus using at least one of the motion sensor and the optical sensor, and obtaining reference information corresponding to the determined activity state.

The at least one operation can include, when determining that the electronic apparatus is normally worn, detecting detachment of the electronic apparatus using at least one of the motion sensor and the optical sensor, and outputting guide information notifying the user of the detachment of the electronic apparatus.

The at least one operation can include, when determining that the electronic apparatus is abnormally worn, outputting guide information notifying the user of the abnormal wearing state.

The at least one operation can include, when determining that the electronic apparatus is abnormally worn, changing setting of the electronic apparatus.

The method can further include communicating with another electronic apparatus, and receiving normal or abnormal wearing information from the another electronic apparatus.

Determining whether the electronic apparatus is normally worn can include determining a wearing direction of the electronic apparatus.

Determining whether the electronic apparatus is normally worn can include obtaining acceleration information for at least one axis of the motion sensor.

The electronic apparatus can include an earbud.

The electronic apparatus and its operating method according to embodiments of the present disclosure can determine the movement based on the information measured by the sensor of the electronic apparatus which is worn on a user's body, output the guide information when the abnormal wearing of the electronic apparatus is detected, and thus guide the user to normally wear the electronic apparatus. Further, the electronic apparatus and its operating method according to embodiments of the present disclosure can change the setting of the electronic apparatus in the abnormal wearing and thus use the same function as the normal wearing of the electronic apparatus.

While the present disclosure has been shown and described with reference to certain embodiments thereof, it will be understood by those skilled in the art that various changes in form and details may be made therein without departing from the spirit and scope of the disclosure as defined by the appended claims and their equivalents.

## Claims

1. An electronic apparatus comprising:
a motion sensor;
an optical sensor;
a memory; and
a processor configured to determine whether the electronic apparatus is worn on a user's body using at least one of the motion sensor and the optical sensor, obtain sensor data according to a motion of the electronic apparatus using at least one of the motion sensor and the optical sensor when determining that the electronic apparatus is worn on the user's body,
compare the obtained sensor data with at least one reference information stored in the memory,
determine whether the electronic apparatus is normally worn based on the comparison, and
perform at least one operation based on the determination.

2. The electronic apparatus of claim 1, wherein the processor is further configured to determine an activity state of the electronic apparatus using at least one of the motion sensor and the optical sensor, and obtain reference information corresponding to the determined activity state.

3. The electronic apparatus of claim 1, wherein, when determining that the electronic apparatus is normally worn, the processor is further configured to detect detachment of the electronic apparatus using at least one of the motion sensor and the optical sensor, and output guide information notifying the user of the detachment of the electronic apparatus.

4. The electronic apparatus of claim 1, wherein, when determining that the electronic apparatus is abnormally worn, the processor is further configured to output guide information notifying the user that the apparatus is abnormally worn.

5. The electronic apparatus of claim 1, wherein, when determining that the electronic apparatus is abnormally worn, the processor is further configured to change a setting of the electronic apparatus.

6. The electronic apparatus of claim 1, further comprising:
a communication unit,
wherein the processor is further configured to communicate with another electronic apparatus through the communication unit, and receive information indicating a normal or abnormal wearing state from the another electronic apparatus.

7. The electronic apparatus of claim 1, wherein the processor is further configured to determine whether the wearing of the electronic apparatus is normal based on a wearing direction of the electronic apparatus.

8. The electronic apparatus of claim 1, wherein the processor is further configured to determine whether the electronic apparatus is normally worn based on acceleration information for at least one axis of the motion sensor.

9. The electronic apparatus of claim 1, wherein the electronic apparatus comprises an earbud.

10. A method for operating an electronic apparatus, comprising:
determining whether the electronic apparatus is worn on a user's body using at least one of a motion sensor and an optical sensor;
obtaining sensor data according to a motion of the electronic apparatus using at least one of the motion sensor and the optical sensor when determining that the electronic apparatus is worn on the user's body;
comparing the obtained sensor data with at least one reference information stored in the electronic device;
determining whether the electronic apparatus is normally worn based on the comparison; and
performing at least one operation based on the determination.

11. The method of claim 10, wherein comparing the obtained data with the at least one reference information comprises:
determining an activity state of the electronic apparatus using at least one of the motion sensor and the optical sensor; and
obtaining reference information corresponding to the determined activity state.

12. The method of claim 10, wherein the at least one operation comprises:
when determining that the electronic apparatus is normally worn, detecting detachment of the electronic apparatus using at least one of the motion sensor and the optical sensor; and
outputting guide information notifying the user of the detachment of the electronic apparatus.

13. The method of claim 10, wherein the at least one operation comprises:
when determining that the electronic apparatus is abnormally worn, outputting guide information notifying the user that the apparatus is abnormally worn.

14. The method of claim 10, wherein the at least one operation comprises:
when determining that the electronic apparatus is abnormally worn, changing a setting of the electronic apparatus.

15. A non-transitory computer-readable recording medium storing a program for determining whether an electronic apparatus is worn on a user's body using at least one of a motion sensor and an optical sensor, obtaining sensor data according to a motion of the electronic apparatus using at least one of the motion sensor and the optical sensor when determining that the electronic apparatus is worn on the user's body, comparing the obtained data with at least one reference information stored in the electronic apparatus, determining whether the electronic apparatus is normally worn based on the comparison, and performing at least one operation based on the determination.
